# DEMANDE DE BREVET EUROPEEN

(11) **EP 4 111 909 A1**
(43) Date de publication de la demande: **04.01.2023**
(21) Numéro de dépôt: 21183464.3
(22) Date de dépôt: 02.07.2021
(51) Int. Cl.: A47C 7/72, A61B 5/00

(54) **SIEGE ERGONOMIQUE**

(71) Demandeur: ERSYS Sàrl, 2900 Porrentruy (CH)
(72) Inventeur: Attouche, Malik, 25160 Montperreux (FR)
(74) Mandataire: AWA Switzerland

(57) **Abrégé**

L'invention concerne un siège ergonomique (1) comprenant un dossier (1a) et une assise (1b) définissant un plan X,Y avec un axe Y de symétrie de l'assise (1b) et avec un axe X perpendiculaire à l'axe Y, l'assise (1b) comprenant au moins 4 capteurs de force (S1 ,S2,S3,S4) avec :
- une première paire de capteurs (S2,S1) disposée de part et d'autre de l'axe Y de symétrie à une distance Y1 de l'axe X avec Y1 compris entre 0 et 130 mm avec un écart X1 entre les capteurs (S2,S1) compris entre 100 et 170 mm,
- une deuxième paire de capteurs (S4,S3) disposée de part et d'autre de l'axe Y de symétrie à une distance Y2 de l'axe X avec Y2 compris entre 185 et 240 mm avec un écart X2 entre les capteurs (S4,S3) compris entre 210 et 265 mm.

La présente invention se rapporte également à la méthode d'optimisation de la posture de l'utilisateur assis sur le siège (1) basée sur les mesures des capteurs.

## Description

### DOMAINE TECHNIQUE

La présente invention se rapporte à un siège ergonomique muni de capteurs. Elle se rapporte également à la méthode permettant d'optimiser la posture de l'utilisateur sur ledit siège en fonction des mesures issues des capteurs.

### ART ANTERIEUR

Selon les offices de statistiques, le taux moyen d'absentéisme au travail s'élève à plusieurs pourcents, ce qui provoque une baisse de productivité considérable. Plusieurs facteurs sont responsables de cet absentéisme. Un des facteurs est l'état de santé du personnel. Les arrêts maladies et accidents de travail représentent à eux seuls plus de 50% de l'absentéisme total. Les motifs d'arrêt de travail sont généralement rattachés à des troubles musculo-squelettiques qui peuvent en grande partie s'expliquer par une mauvaise posture de travail récurrente et prolongée.

Pour améliorer la posture des utilisateurs, il a été proposé dans l'art antérieur des sièges munis de capteurs qui permettent sur base des mesures issues de ces capteurs de fournir aux utilisateurs des recommandations pour un meilleur maintien sur le siège.

Ainsi, le document EP 3 788 914 divulgue un siège ergonomique avec six capteurs, deux sur l'assise et quatre sur le dossier. Les capteurs dans le dossier sont des capteurs de distance et de rotation tandis que les capteurs dans l'assise sont des capteurs de force. Le siège est muni d'un microcontrôleur qui analyse les données en temps réel pour ensuite conseiller l'utilisateur sur sa posture.

Ces sièges ergonomiques connectés représentent une avancée considérable dans le domaine de la prévention santé. Cependant, la position, le nombre et le type des capteurs à placer dans le siège sont encore sujet à optimisation pour une meilleure posture de l'utilisateur sur le siège.

### RESUME DE L'INVENTION

La présente invention a pour objet de proposer un nouveau siège muni d'au moins quatre capteurs de force localisés dans des zones précises de l'assise. De préférence, ce siège comporte également sur le dossier au moins un capteur de distance.

Plus précisément, l'invention se rapporte à un siège ergonomique comprenant un dossier et une assise présentant une partie arrière faisant face au dossier et une partie avant opposée à ladite partie arrière, l'assise définissant un plan X,Y avec un axe Y de symétrie de l'assise s'étendant entre la partie arrière et la partie avant de l'assise et avec un axe X perpendiculaire à l'axe Y et passant par une extrémité de la partie arrière de l'assise, le dossier étant défini par un axe Z de symétrie perpendiculaire au plan X,Y, ladite assise comprenant au moins 4 capteurs de force avec :
- une première paire de capteurs disposée de part et d'autre de l'axe Y de symétrie à une distance Y1 de l'axe X avec Y1 compris entre 0 et 130 mm, l'écart X1 entre les capteurs de la première paire de capteurs étant compris entre 100 et 170 mm, et
- une deuxième paire de capteurs disposée de part et d'autre de l'axe Y de symétrie à une distance Y2 de l'axe X avec Y2 compris entre 185 et 240 mm, l'écart X2 entre les capteurs de la deuxième paire de capteurs étant compris entre 210 et 265 mm.

De préférence, le dossier comprend au moins un capteur de distance destiné à mesurer la distance entre le dossier et le buste d'un utilisateur assis sur le siège, le capteur de distance étant positionné sur l'axe Z.

De préférence, la partie arrière de l'assise présente un dégagement au niveau de l'origine des axes X et Y permettant de libérer le coccyx de l'utilisateur. En combinaison avec les données issues des capteurs, le dégagement au niveau du coccyx permet d'optimiser la répartition de la pression sur l'assise du siège.

La présente invention se rapporte également à une méthode d'optimisation de la posture d'un utilisateur assis sur le siège ergonomique décrit ci-avant, ladite méthode comprenant les étapes suivantes :
- calcul de la position du centre de gravité (G(xg,yg)) de l'utilisateur dans le plan X,Y sur base des mesures des capteurs de force situés dans l'assise ;
- rectification de la posture de l'utilisateur si le centre de gravité (G(xg,yg)) se trouve en dehors d'une zone donnée ;
- calcul de la position du barycentre (B(xb,yb)) de l'utilisateur projeté dans le plan X,Y sur base de la position du centre de gravité (G(xg,yg)), de la mesure éventuelle du capteur de distance, des mesures éventuelles de force issues du ou des capteurs de force du dossier et d'un coefficient α qui est fonction de la morphologie de l'utilisateur ;
- calcul de la distance dans le plan X,Y entre le centre de gravité (G(xg,yg)) et le barycentre (B(xb,yb)),
- rectification de la posture de l'utilisateur si cette distance est supérieure à une valeur donnée.

La méthode selon l'invention permet ainsi de :
- déterminer la répartition du poids du corps sur l'assise et le dossier,
- stabiliser une assise fémoro-ischiatique (cuisse, ischion),
- décharger l'appui du coccyx,
- favoriser le maintien lombaire sur le dossier,
- gérer le centre de gravité du poids sur l'assise (G),
- gérer les écarts minimums entre le centre de gravité (G) et le barycentre du corps (B) projeté sur l'assise,
- informer en temps réel la personne assise d'une mauvaise/bonne posture,
- proposer des changements de postures et des étirements en fonction du temps statique pour éviter les douleurs et les ankyloses,
- auto-former la personne assise.

D'autres caractéristiques et avantages de la présente invention apparaîtront dans la description suivante d'un mode de réalisation préféré, présenté à titre d'exemple non limitatif en référence aux dessins annexés.

### BREVE DESCRIPTION DES FIGURES

La figure 1 représente schématiquement le siège ergonomique selon l'invention muni des capteurs dans l'assise et dans le dossier.
La figure 2 représente une vue en plan de l'assise du siège ergonomique avec le positionnement des capteurs de force.
La figure 3 représente une vue en plan du dossier du siège ergonomique avec le positionnement d'un capteur de distance.
La figure 4 représente le logigramme de calcul du centre de gravité, du barycentre et du calcul de l'écart entre ces derniers menant à la détermination de la bonne posture de l'utilisateur.
La figure 5 représente schématiquement les différentes couches de mousse sur le support du dossier ou de l'assise.

### DESCRIPTION DETAILLEE

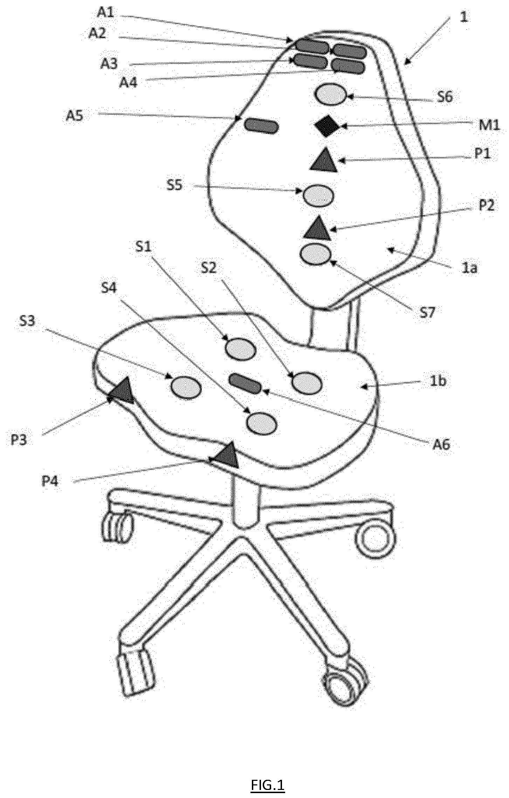

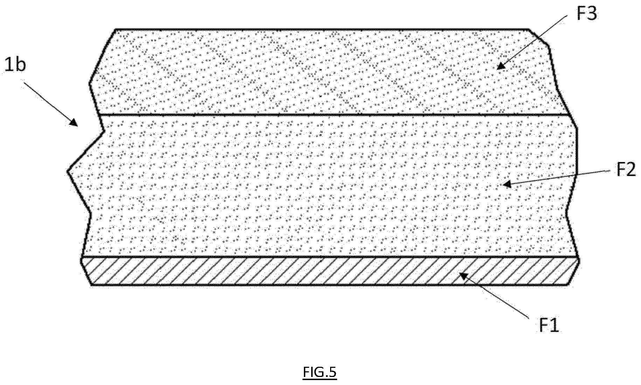

La présente invention se rapporte à un siège ergonomique muni de capteurs. Ledit siège 1 est représenté à la figure 1 avec un ensemble de capteurs dont certains sont facultatifs, répartis dans l'assise 1b et dans le dossier 1a. Ces capteurs sont fixés sur un support, préférentiellement rigide, par exemple réalisé en bois, polymère, fibre de carbone, alliage métallique, etc. Le support F1 représenté à la figure 5 est surmonté d'une ou plusieurs couches de mousse F2,F3. De préférence, le support est surmonté de deux couches de mousse. Les capteurs sont disposés entre le support F1 et la première couche de mousse F2 ou éventuellement entre deux couches de mousse.

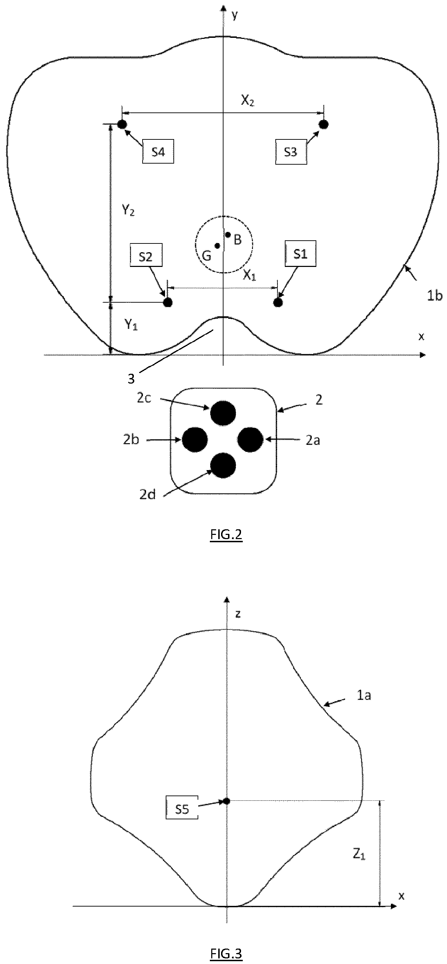

L'assise 1b du siège 1 représentée à la figure 2 comporte au moins quatre capteurs de force (S1,S2,S3,S4) disposés à des endroits bien particuliers. Il s'agit de capteurs de force avec une mesure en N (Newton), cette dernière étant ensuite convertie en une pression (Newton/m²). Leur position est donnée par rapport à un système d'axes X et Y. Ainsi, l'assise définit un plan X,Y avec un axe Y qui s'étend entre la partie arrière et la partie avant de l'assise. Cet axe Y forme l'axe de symétrie de l'assise avec respectivement deux capteurs de force disposés de chaque côté de l'axe Y en vis-à-vis. Perpendiculairement à cet axe Y, s'étend l'axe X sur l'extrémité de la partie arrière de l'assise.

Pour permettre le calcul du centre de gravité G de l'utilisateur assis sur le siège, ces 4 capteurs doivent être positionnés précisément à des endroits donnés qui ont été déterminés sur base d'une campagne d'essais. La position des capteurs est donnée par quatre distances X1,X2,Y1,Y2, les distances étant mesurées par rapport au centre du capteur. X1 est la distance entre les capteurs S1 et S2 qui sont disposés de chaque côté de l'axe Y de symétrie. X1 doit être compris entre 100 et 170 mm, de préférence entre 115 et 145 mm, plus préférentiellement entre 120 et 140 mm. X2 est la distance entre les capteurs S3 et S4 qui sont disposés de chaque côté de l'axe Y de symétrie à proximité de la partie avant de l'assise. X2 doit être compris entre 210 et 265 mm, de préférence entre 220 et 255 mm, plus préférentiellement entre 230 et 245 mm. Y1 qui est la distance entre la paire de capteurs S1,S2 et l'axe X doit être comprise entre 0 et 130 mm, de préférence entre 30 et 90 mm, plus préférentiellement entre 50 et 70 mm. Y2 qui est la distance entre la paire de capteurs S1,S2 et la paire de capteurs S3,S4 doit être comprise entre 185 et 240 mm, de préférence entre 195 et 230 mm, plus préférentiellement entre 205 et 220 mm.

A titre d'exemple, les capteurs de force peuvent être des capteurs résistifs tels que des films ou des boutons. De manière moins avantageuse, les 4 capteurs distincts pourraient être remplacés par une nappe de capteurs de pression positionnés sur l'assise avec au moins 4 capteurs positionnés comme indiqué précédemment.

Avantageusement, l'assise 1b comporte également des capteurs de position P3,P4 qui mesurent la hauteur et l'inclinaison de l'assise. Par exemple, ces capteurs peuvent être positionnés sur la partie avant de l'assise (fig.1). Elle peut également comporter au moins un capteur dit d'ambiance A6, ce capteur peut mesurer le niveau sonore, la luminosité, la température, l'hygrométrie, le flux et la vitesse de l'air, la température de contact au niveau de l'assise, ou une éventuelle vibration.

De préférence, le dossier 1a représenté aux figures 1 et 3 comporte au moins un capteur de distance S5 qui mesure la distance entre le dossier 1a et le buste de l'utilisateur.

Le dossier 1a définit un plan avec un axe X correspondant à l'axe X de l'assise et un axe Z perpendiculaire au plan X,Y et s'étendant entre la partie inférieure et la partie supérieure du dossier. L'axe Z forme un axe de symétrie du dossier. Préférentiellement, le capteur S5 est disposé sur cet axe Z à une distance Z1 de l'axe X comprise entre 350 et 500 mm, de préférence entre 400 et 450 mm. Cette distance par rapport à l'axe X prend en compte le fait que le dossier peut être réglable en hauteur par rapport à l'assise, l'axe X sur l'assise étant le référentiel.

Préférentiellement, le dossier 1a comporte également au moins un capteur de force. Il peut ainsi comporter un capteur de force S6, deux capteurs de force S6,S7 ou plus de deux capteurs de force dans le dossier 1a (fig.1). Ces capteurs sont préférentiellement situés sur l'axe Z à une distance de l'axe X comprise entre 150 et 250 mm et entre 350 et 450 mm pour respectivement le capteur S6 et le capteur S7. Cette distance par rapport à l'axe X prend également en compte le fait que le dossier peut être réglable en hauteur par rapport à l'assise, l'axe X sur l'assise étant le référentiel.

Optionnellement, le dossier 1a peut comporter un ou plusieurs capteurs de position. Dans l'exemple illustré à la figure 1, le dossier 1a comporte deux capteurs de position P1,P2 pour mesurer respectivement la hauteur de l'appui lombaire P2 et l'inclinaison du dossier P1. Préférentiellement, ils sont situés sur l'axe Z. Optionnellement, le dossier 1a peut également comporter un ou plusieurs capteurs d'ambiance, référencés A1 à A5 dans la figure 1. Ces capteurs peuvent mesurer le niveau sonore, la luminosité, la température, l'hygrométrie, le flux et la vitesse de l'air, la température de contact au niveau du dossier, ou une éventuelle vibration. Toujours à titre optionnel, le dossier 1a peut comporter un capteur de mouvement M1 qui mesure l'accélération, la fréquence et la durée des accélérations. Préférentiellement, il est positionné sur l'axe Z.

Comme susmentionné, les capteurs de force, et de manière générale, l'ensemble des capteurs sont situés entre le support F1 et la première couche de mousse F2 (fig.5) ou éventuellement entre deux couches de mousse. Pour une mesure optimale de la force, la masse volumique et l'épaisseur de la mousse doivent être bien choisies. De préférence, l'ensemble avec une ou plusieurs couches a une épaisseur totale pour l'assise, et éventuellement pour le dossier si il comporte un capteur de force, comprise entre 40 et 120 mm, plus préférentiellement entre 60 et 100 mm et encore plus préférentiellement entre 70 et 90 mm. De préférence, la masse volumique est comprise entre 40 et 60 kg/m³, plus préférentiellement entre 45 et 55 kg/m³. Avantageusement, l'assise et le dossier comportent deux couches de mousse avec une couche extérieure, c.à.d. une deuxième couche, réalisée dans une mousse à mémoire de forme viscoélastique pour le confort de l'utilisateur.

Le siège comporte également, de préférence dans l'assise, un microcontrôleur (non représenté) avec le logiciel de calcul, entre autres, du barycentre et du centre de gravité, un transmetteur/récepteur de données qui communique avec un boîtier 2 muni de moyens signalant à l'utilisateur sa bonne ou mauvaise posture en fonction des mesures réalisées avec les capteurs de force et éventuellement des capteurs de distance et de position (fig.2). Ce boîtier 2 peut être positionné sur le poste de travail, sur le siège ou ailleurs pour autant qu'il soit visible pour l'utilisateur. Par exemple, le boîtier 2 peut comporter des led's 2a,2b,2c,2d qui respectivement peuvent indiquer une bonne ou mauvaise posture via quatre led's de couleurs distinctes. En alternative, il pourrait s'agir d'un écran. Le siège muni des capteurs et des moyens informatiques peut être alimenté par batterie ou via le secteur.

De préférence, la partie arrière de l'assise 1b présente un dégagement 3 au niveau de l'origine des axes X et Y permettant de libérer le coccyx de l'utilisateur. Ce design permet d'obtenir une meilleure répartition de la pression exercée par l'utilisateur sur l'assise et donc une meilleure posture.

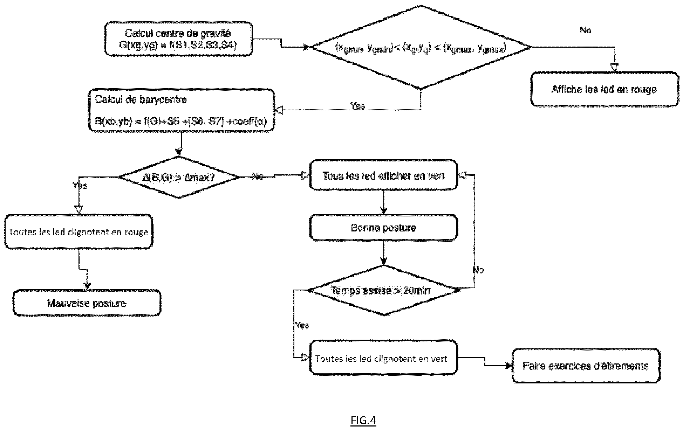

La présente invention se rapporte également à la méthode permettant d'améliorer la posture de l'utilisateur en position assise sur le siège. La méthode est basée sur le calcul du centre de gravité G de l'utilisateur sur l'assise et du barycentre B qui correspond au centre de gravité du corps de l'utilisateur projeté sur l'assise. Selon l'invention, l'écart entre G et B doit être minimisé pour une posture idéale de l'utilisateur. Le logigramme à la figure 4 schématise les étapes mises en œuvre lors de la méthode d'optimisation.

Une première étape consiste à déterminer la position du centre de gravité sur l'assise selon les axes X et Y (G(xg,yg)) sur base des mesures des 4 capteurs de force de l'assise (S1,S2,S3,S4). Le centre de gravité G doit se situer dans une région donnée qui est proche ou sur l'axe Y de symétrie ; cette région est schématiquement (pas à l'échelle) représentée à la figure 2. De préférence, l'écart entre G et l'axe Y, c.à.d. la coordonnée yg, est inférieur ou égal à 50 mm et l'écart entre G et l'axe X, c.à.d. la coordonnée xg, est compris entre 60 et 170 mm. Si G se trouve en dehors de cette région, une ou plusieurs led's s'affichent en rouge, ce qui amène l'utilisateur à modifier sa position. Si G se trouve dans cette région, une deuxième étape de la méthode consiste à calculer la position du barycentre B (B(xb,yb)) projeté dans le plan X,Y. Sa position est calculée sur base de la position du centre de gravité, de la mesure éventuelle de distance issue du capteur S5 si le dossier est muni d'un tel capteur, des mesures éventuelles de force issues du ou des capteurs S6,S7 et d'un coefficient α qui est fonction de la morphologie de l'utilisateur. Ensuite, dans une troisième étape, la distance entre B et G est calculée. Cette distance doit être minimisée. De préférence, la distance entre B et G selon la direction de l'axe X est inférieure ou égale à 50 mm et la distance selon la direction de l'axe Y est inférieure ou égale à 100 mm. Idéalement, G et B sont situés tous deux sur l'axe Y à faible distance.

Si l'écart maximal n'est pas respecté, l'utilisateur est amené à changer sa position. Dans le cas contraire, l'affichage LED ou l'écran indique à l'utilisateur que sa posture est idéale.

La méthode comporte ensuite optionnellement des étapes de calcul du temps d'assise et d'émissions de recommandations pour réaliser des exercices d'étirement. La méthode comporte également optionnellement une étape de traitement et d'analyse de données à des fins statistiques pour la prévention santé.

Selon l'invention, la présence de capteurs de force de chaque côté de l'axe de symétrie Y permet également de déterminer si la répartition du poids de l'utilisateur est symétrique sur l'assise. De même, les capteurs de force positionnés à l'avant et à l'arrière permettent de calculer la répartition du poids entre l'avant et l'arrière de l'utilisateur sur l'assise.

Pour finir, on précisera que le siège ergonomique selon l'invention peut aisément s'intégrer dans le poste de travail ergonomique pour horloger selon le document EP 3 117 965.

### Légende

(1) Siège ergonomique
   a. Dossier
   b. Assise
(2) Boîtier ou dispositif d'affichage a,b,c,d: LED
(3) Dégagement
   F1 : Support
   F2 : Première couche de mousse
   F3 : Deuxième couche de mousse
   S1,S2,S3,S4,S6,S7 : Capteurs de force
   S5: Capteur de distance
   G : Centre de gravité sur l'assise
   B : Barycentre du corps projeté sur l'assise
   A1 à A6 : Capteurs d'ambiance
   M1 : Capteur de mouvement
   P1 à P4 : Capteurs de position

## Revendications

1. Siège ergonomique (1) comprenant un dossier (1a) et une assise (1b) présentant une partie arrière faisant face au dossier (1a) et une partie avant opposée à ladite partie arrière, l'assise (1b) définissant un plan X,Y avec un axe Y de symétrie de l'assise (1b) s'étendant entre la partie arrière et la partie avant de l'assise (1b) et avec un axe X perpendiculaire à l'axe Y et passant par une extrémité de la partie arrière de l'assise (1b), le dossier (1a) étant défini par un axe Z de symétrie perpendiculaire au plan X,Y, ladite assise (1b) comprenant au moins 4 capteurs de force (S1,S2,S3,S4) avec :
- une première paire de capteurs (S2,S1) disposée de part et d'autre de l'axe Y de symétrie à une distance Y1 de l'axe X avec Y1 compris entre 0 et 130 mm, l'écart X1 entre les capteurs (S2,S1) de la première paire de capteurs (S2,S1) étant compris entre 100 et 170 mm et
- une deuxième paire de capteurs (S4,S3) disposée de part et d'autre de l'axe Y de symétrie à une distance Y2 de l'axe X avec Y2 compris entre 185 et 240 mm, l'écart X2 entre les capteurs (S4,S3) de la deuxième paire de capteurs (S4,S3) étant compris entre 210 et 265 mm.

2. Siège ergonomique (1) selon la revendication précédente, **caractérisé en ce que** ledit dossier (1a) comprenant au moins un capteur de distance (S5) destiné à mesurer la distance entre le dossier (1a) et le buste d'un utilisateur assis sur le siège (1), le capteur de distance (S5) étant positionné sur l'axe Z.

3. Siège ergonomique (1) selon l'une des revendications précédentes, **caractérisé en ce que** Y1 est compris entre 30 et 90 mm, de préférence entre 50 et 70 mm.

4. Siège ergonomique (1) selon l'une des revendications précédentes, **caractérisé en ce que** X1 est compris entre 115 et 145 mm, de préférence entre 120 et 140 mm.

5. Siège ergonomique (1) selon l'une des revendications précédentes, **caractérisé en ce que** Y2 est compris entre 195 et 230 mm, de préférence entre 205 et 220 mm.

6. Siège ergonomique (1) selon l'une des revendications précédentes, **caractérisé en ce que** X2 est compris entre 220 et 255 mm, de préférence entre 230 et 245 mm.

7. Siège ergonomique (1) selon l'une des revendications précédentes, **caractérisé en ce que** la partie arrière de l'assise (1b) présente un dégagement (3) au niveau de l'origine des axes X et Y permettant de libérer le coccyx de l'utilisateur.

8. Siège ergonomique (1) selon l'une des revendications précédentes, **caractérisé en ce que** l'assise (1b) et éventuellement le dossier (1a) comporte un support (F1) et une ou plusieurs couches de mousse (F2,F3) avec une première couche de mousse (F2) en contact avec le support (F1), la ou les couches ayant une épaisseur totale comprise entre 40 et 120 mm, de préférence entre 60 et 100 mm et plus préférentiellement entre 70 et 90 mm.

9. Siège ergonomique (1) selon la revendication précédente, **caractérisé en ce que** chaque couche de mousse a une masse volumique comprise entre 25 et 65 kg/m³, de préférence entre 45 et 55 kg/m³.

10. Siège ergonomique (1) selon l'une des revendications précédentes, **caractérisé en ce que** l'assise (1b) et/ou le dossier (1a) comporte :
- au moins deux capteurs de position (P1,P2,P3,P4), et/ou
- un ou plusieurs capteurs d'ambiance (A1,A2,A3,A4,A5,A6) pour mesurer respectivement un niveau sonore, une luminosité, une température, une hygrométrie, un flux et une vitesse de l'air, une température de contact au niveau de l'assise (1b) et/ou du dossier (1a), ou une vibration.

11. Siège ergonomique (1) selon l'une des revendications précédentes, **caractérisé en ce que** le dossier (1a) comporte :
- un capteur de mouvement (M1) qui mesure l'accélération, la fréquence et la durée des accélérations subies par le dossier (1a), et/ou
- au moins un capteur de force (S6).

12. Ensemble comprenant le siège ergonomique (1) selon l'une des revendications précédentes et un dispositif d'affichage (2) permettant à l'utilisateur d'adapter sa posture.

13. Méthode d'optimisation de la posture d'un utilisateur assis sur le siège ergonomique (1) selon l'une des revendications 1 à 11, ladite méthode comprenant les étapes suivantes :
- calcul de la position du centre de gravité (G(xg,yg)) de l'utilisateur dans le plan X,Y sur base des mesures des capteurs de force (S1,S2,S3,S4) situés dans l'assise (1b) ;
- rectification de la posture de l'utilisateur si le centre de gravité (G(xg,yg)) se trouve en dehors d'une zone donnée ;
- calcul de la position du barycentre (B(xb,yb)) de l'utilisateur projeté dans le plan X,Y sur base de la position du centre de gravité (G(xg,yg)), de la mesure éventuelle du capteur de distance (S5), des mesures éventuelles de force issues du ou des capteurs de force (S6,S7) du dossier (1a) et d'un coefficient α qui est fonction de la morphologie de l'utilisateur ;
- calcul de la distance dans le plan X,Y entre le centre de gravité (G(xg,yg)) et le barycentre (B(xb,yb)),
- rectification de la posture de l'utilisateur si cette distance est supérieure à une valeur donnée.

14. Méthode selon la revendication précédente, **caractérisée en ce que**, dans la zone donnée, l'écart entre G et l'axe Y (yg) est inférieur ou égal à 50 mm, **en ce que** l'écart entre G et l'axe X (xg) est compris entre 60 et 170 mm et **en ce que** la valeur donnée selon la direction de l'axe X est inférieure ou égale à 50 mm et selon la direction de l'axe Y est inférieure ou égale à 100 mm.

15. Méthode selon la revendication 13 ou 14, **caractérisée en ce qu'**elle comporte une étape de traitement et d'analyse des données à des fins statistiques pour la prévention santé.
